# EUROPEAN PATENT SPECIFICATION

(11) **EP 3 192 564 B1**
(45) Date of publication and mention of the grant of the patent: **10.07.2024**
(21) Application number: 16150964.1
(22) Date of filing: 12.01.2016
(51) Int. Cl.: A61Q 11/00, A61K 8/66, A61K 8/73

(54) **ORAL CARE COMPOSITION COMPRISING A PROTEIN FOR BALANCING THE BACTERIAL FLORA OF THE MOUTH**
MUNDPFLEGEZUSAMMENSETZUNG
COMPOSITION DE PROTECTION ORALE

(43) Date of publication of application: 19.07.2017
(73) Proprietor: Unilever IP Holdings B.V., 3013 AL Rotterdam (NL); Unilever Global IP Limited, Wirral, Merseyside CH62 4ZD (GB)
(72) Inventor: Adams, Suzanne Elizabeth, Bebington, Wirral, Merseyside CH63 3JW (GB); Arnold, David Stanley, Bebington, Wirral, Merseyside CH63 3JW (GB); Brading, Melanie Gayle, Bebington, Wirral, Merseyside CH63 3JW (GB); Murphy, Barry, Bebington, Wirral, Merseyside CH63 3JW (GB)
(74) Representative: Tansley, Sally Elizabeth

(56) References cited:
- EP-A1- 1 334 710
- WO-A1-88/02600
- WO-A1-94/05251
- CN-A- 101 721 324
- CN-A- 104 188 860
- JP-A- 2002 322 088
- US-A- 4 178 362
- US-A- 4 537 764
- ANONYMOUS: "Lysozyme - Wikipedia", 14 September 2023 (2023-09-14), XP093082432, Retrieved from the Internet <URL:https://en.wikipedia.org/wiki/Lysozyme> [retrieved on 20230914]

## Description

### Field of the Invention

The present invention is concerned with oral care compositions. More particularly, the present invention is concerned with oral care compositions containing enzymes.

### Background of the Invention

Conventional thinking with respect to toothpastes is that anti-bacterials are needed to destroy the bacteria within the mouth. The present application has found that it is beneficial to adjust the balance away from bacteria associated with poor oral health in favour of those associated with good oral health.

WO 88/02600 A1 discloses bactericidal compositions comprising glucose oxidase, lactoperoxidase and lysozyme.

The present application relates to compositions that balance the bacterial flora of the mouth.

### Description of the Invention

The present invention relates to a composition comprising a zinc ion and enzymes for use in balancing the bacterial flora of the mouth and promoting oral health as defined in claim 1.

### Detailed Description of the Invention

The oral microbiome is highly complex and diverse community with over 700 different species having been identified. In general health related bacteria are facultative anaerobic or aerobic bacteria (e.g genus Neisseria) and bacteria related to gum disease are anaerobic bacteria (e.g genus Treponema). There is therefore a need for the promotion of aerobic bacteria in the mouth and a decrease in the number of anaerobic bacteria.

The present application has found that compositions comprising enzymes can be used to decrease the numbers of anaerobic bacterial relative to the abundance of the aerobic bacteria.

The enzymes are selected from the following groups:
i) a glycoside hydrolase, specifically amylglucosidase;
ii) an oxoreductase acting on OH groups of donors, specifically glucose oxidase;
iii) a hemeperoxidase, specifically a lactoperoxidase.

The enzymes comprise a mixture of amylglucosidase, glucose oxidase and lactoperoxidase.

Preferably the total level of enzyme is from 0.01 wt% to 2wt% of the total composition. More preferably from 0.1 wt% to 1 wt% of the total composition.

Preferably the total level of glycoside hydrolase in the composition is from 0.05 to 1 wt% of the total composition.

Preferably the total level of oxoreductase acting on OH groups of donors is from 0.05 to 1 wt% of the total composition.

Preferably the total level of hemeperoxidase is from 0.0005 to 0.01 wt% of the total composition.

Preferably the weight ratio of glycoside hydrolase to other enzymes in the composition is greater than 1:1, more preferably greater than 3:2.

Compositions for use of the invention may also comprise other proteins such as lysozymes, lactoferrins and colostrum. Preferably these other proteins are present at total levels from 0.001 to 0.5 wt% of the total composition, preferably from 0.005 to 0.1 wt% of the total composition.

Furthermore, the oral care composition will usually contain a surfactant in an amount of from 0.2 to 5% by weight based on the total weight of the dentifrice. Preferred are nonionic surfactants such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Particularly preferred are polyethylene glycol ethers of fatty alcohols in particular polyethylene glycol ethers having from 20 to 40 ethylene oxide groups per unit. Examples of such suitable surfactant include the group of surfactants known as Steareth surfactants such as Steareth 30. Preferably the level of non-ionic surfactant is from 0.5 to 7 wt% of the total composition, more preferably from 1 to 5 wt% of the total composition

Although other surfactants may be present it is preferred if they are limited to levels below 0.5 wt% of the total composition preferably less than 0.1 wt% of the total composition.

The oral care composition, especially in the cases of dentifrices, may also contain structurants and agents such as binders and thickening agents. These structurants will generally be present in an amount of from 0.1 to 1% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth). Natural gum based thickeners, in particular carrageenan are particularly preferred.

Examples of suitable product forms for compositions of the invention include dentifrices, mouthwashes, mouthsprays and liquid formulations.

Preferred product forms for compositions for use of the invention are those which are suitable for brushing and/or rinsing the surfaces of the oral cavity. Dentifrices/toothpastes are preferred.

In such preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

An example of a preferred type of product form in the context of the present invention is a dentifrice.

The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition for use according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolysed polysaccharides and mixtures thereof. Glycerol and sorbitol are preferred, sorbitol being particularly preferred.

The oral care composition, in particular dentifrice compositions may comprise abrasive materials.

Abrasive materials will generally be present in an amount of from 0.5 to 75%, preferably 3 to 60% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred.

Compositions for use of the invention may also comprise thickeners such as finely divided silicas, hectorites, calcium carbonate, colloidal magnesium aluminium silicates and mixtures thereof.

Compositions for use of the invention comprise a zinc ion. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

Compositions for use of the present invention may also contain further optional ingredients customary in the art such as fluoride ion sources, anticalculus agents, buffers, flavouring agents, sweetening agents, colouring agents, opacifying agents, preservatives, antisensitivity agents, bleaching agents, and antimicrobial agents.

To clean the surfaces of the oral cavity, the composition for use according to the invention is topically applied to the oral cavity surfaces to be cleaned.

Preferably the composition for use according to the invention is topically applied to the oral cavity surfaces to be cleaned and then agitated by brushing and/or rinsing.

The invention will now be illustrated by the following non-limiting Examples:

### Examples

A double-blind, randomised, parallel study to collect plaque samples was conducted by an independent research organization. Samples from 102 subjects completed the microbiomics analysis, 52 using Example 1 and 50 using standard silica SLS toothpaste - Example A. After recruitment, subjects used a silica SLS (Example A) toothpaste for up to four weeks, and then subjects used their allocated test product at home, twice a day for 14 weeks. Samples of plaque were collected at baseline and at the end of the 14 week brushing period for microbiomic analysis. Study Products:

### Example 1

| **Material** | **Amount % W/W** |
|---|---|
| Sorbitol | 28.5 |
| Zinc Gluconate | 0.3 |
| Sodium Fluoride | 0.3 |
| Citric Acid | 0.3 |
| Disodium Phosphate | 0.5 |
| Sodium Caseinate? | 0.01 |
| Titanium Dioxide | 0.5 |
| Zeodent 113* | 16.0 |
| Tixosil 43** | 4.8 |
| Carrageenan | 0.7 |
| Glycerin | 5.0 |
| Xanthum gum | 1.0 |
| Steareth 30 | 3.0 |
| Amylglucosidase | 0.3 |
| Lactoperoxidase | 0.002 |
| Lysozyme (22%)*** | 0.05 |
| Glucose Oxidase | 0.14 |
| Lactoferrin | 0.01 |
| Colostrum | 0.01 |
| Water and minors | to100.0 |

| | |
|---|---|
| (composition contains 1450ppm F as NaF) * Precipitated silica ** Thickening silica *** 0.495% lysozyme liquid at 22% lysozyme | |

### Example A

| **Material** | **Amount (%W/W)** |
|---|---|
| Sorbitol 70/70 | 45 |
| PEG-32 | 5 |
| Monosodium Phosphate | 0.1 |
| Sodium Fluoride | 0.32 |
| Titanium Dioxide | 1 |
| Tixosil 43* | 7.5 |
| Sorbosil AC77** | 10 |
| Cellulose gum | 0.63 |
| Sodium Lauryl Sulphate | 1.5 |
| Water and minors | To 100 |

| | |
|---|---|
| Silica SLS Toothpaste (containing 1450ppm F as NaF) • hydrated thickening silica ** hydrated silica | |

### Microbiomics analysis:

Plaque samples were processed and sequence data were generated based on the V4-V6 region of the 16S rRNA gene using the latest Illumina sequencing techniques. Species level identification was carried out using the Human Oral Microbiome Database (HOMD). The data was analysed using a range of recognised statistical tests designed for microbiomics.

### Results:

Following statistical analysis (PERMANOVA) the following changes were identified:
- No significant difference was observed between the population for Example 1 and Example A at baseline (p=0.26).
- There was a significant shift in population for Example 1 from baseline to 14 weeks (p=0.04).
- In contrast, for Example A, no change in population was seen from baseline to 14 weeks (p=0.99).
- A comparison of the populations at the 14 week time point showed a significant difference between the two products (p=0.01).

Detailed analysis of the species over time showed a significant increase in health related aerobic bacteria (belonging to the genus Neisseria) was observed with a concomitant decrease in disease related anaerobic bacteria (belonging to the genus Treponema) in Example 1. No such overall changes were observed for Example A.

## Claims

1. A composition comprising a zinc ion and enzymes for use in balancing the bacterial flora of the mouth and promoting oral health by decreasing the numbers of anaerobic bacterial relative to the abundance of the aerobic bacteria in the mouth in which the enzymes comprise a mixture of amylglucosidase, glucose oxidase and lactoperoxidase.

2. A composition for use according to any preceding claim in which the composition further comprises a non-ionic surfactant.

3. A composition for use according to claim 2 in which the non-ionic surfactant is a polyethylene glycol ethers of a fatty alcohol.

4. A composition for use according to claim 3 in which the non-ionic surfactant is Steareth 30.

5. A composition for use according to any preceding claim in which the composition further comprises a further protein.

6. A composition for use according to any preceding claim in which the composition comprises a carrageenan based thickener.

## Patentansprüche

1. Zusammensetzung, umfassend ein Zinkion und Enzyme zur Verwendung beim Ausbalancieren der Bakterienflora des Munds und zur Förderung der Mundgesundheit durch Verringerung der Anzahl anaerober Bakterien in Bezug auf den Überfluss der aeroben Bakterien im Mund, in welchem die Enzyme eine Mischung von Amylglucosidase, Glucoseoxidase und Lactoperoxidase umfassen.

2. Zusammensetzung zur Verwendung nach vorhergehendem Anspruch, wobei die Zusammensetzung ferner ein nichtionisches Tensid umfasst.

3. Zusammensetzung zur Verwendung nach Anspruch 2, wobei das nichtionische Tensid ein Polyethylenglycolether eines Fettalkohols ist.

4. Zusammensetzung zur Verwendung nach Anspruch 3, wobei das nichtionische Tensid Steareth 30 ist.

5. Zusammensetzung zur Verwendung nach einem vorhergehenden Anspruch, wobei die Zusammensetzung ferner ein weiteres Protein umfasst.

6. Zusammensetzung zur Verwendung nach einem vorhergehenden Anspruch, wobei die Zusammensetzung ein Verdickungsmittel auf Carrageen-Basis umfasst.

## Revendications

1. Composition comprenant un ion zinc et des enzymes destinée à être utilisée pour équilibrer la flore bactérienne de la bouche et favoriser la santé bucco-dentaire en diminuant le nombre de bactéries anaérobies par rapport à l'abondance des bactéries aérobies dans la bouche, où les enzymes comprennent un mélange d'amylglucosidase, de glucose oxydase et de lactoperoxydase.

2. Composition destinée à être utilisée selon l'une quelconque des revendications précédentes, où la composition comprend en outre un tensioactif non ionique.

3. Composition destinée à être utilisée selon la revendication 2, où le tensioactif non ionique est un éther de polyéthylèneglycol d'un alcool gras.

4. Composition destinée à être utilisée selon la revendication 3 où le tensioactif non ionique est le Steareth 30.

5. Composition destinée à être utilisée selon l'une quelconque des revendications précédentes, où la composition comprend en outre une autre protéine.

6. Composition destinée à être utilisée selon l'une quelconque des revendications précédentes, où la composition comprend un épaississant à base de carraghénane.
